# EUROPÄISCHE PATENTSCHRIFT

(11) **EP 1 515 732 B1**
(45) Veröffentlichungstag und Bekanntmachung des Hinweises auf die Patenterteilung: **14.01.2009**
(21) Anmeldenummer: 03759935.4
(22) Anmeldetag: 12.06.2003
(51) Int. Cl.: A61K 33/00, A61P 25/00

(54) **CEREBRALE PROTEKTION MIT EINEM XENONHALTIGEN GAS**
CEREBRAL PROTECTION WITH A GAS COMPRISING XENON
PROTECTION CEREBRALE A L'AIDE D'UN GAZ CONTENANT DU XENON

(30) Priorität: 12.06.2002 DE 10226191; 22.06.2002 DE 10227975; 10.08.2002 DE 10236765
(43) Veröffentlichungstag der Anmeldung: 23.03.2005
(73) Patentinhaber: Air Liquide Deutschland GmbH, 40235 Düsseldorf (DE)
(72) Erfinder: NEU, Peter, 47228 Duisburg (DE); PILGER, Carsten, 47509 Rheurdt (DE); REYLE-HAHN, Matthias, 14129 Berlin (DE)
(74) Vertreter: Kahlhöfer, Hermann
(86) Internationale Anmeldenummer: PCT/EP2003/006157
(87) Internationale Veröffentlichungsnummer: WO 2003/105871

(56) Entgegenhaltungen:
- WO-A-00/53192
- WO-A-02/09731
- WO-A-02/22141
- WO-A-97/15311
- WO-A-02/078863
- DE-A- 19 933 704

## Beschreibung

Die Erfindung betrifft die Verwendung, von Yen on oder eines Xenon-haltigen Gasgemisches.

In der WO 02/22141 A2 wird der Einsatz von Xenon oder xenonhaftigen Gasen als Arzneimittel, insbesondere Herz-Kreislaufmittel, beschrieben.

In der DE 19933704 A1 wird die Verwendung einer flüssigen Präparation, die ein lipophiles Gas wie Xenon enthält, zur Neuroprotektion und Neuroregeneration beschrieben.

Bei der Neuroprotektion und Neuroregeneration geht es um den Schutz und die Regeneration einzelner Nervenzellen durch die Einwirkung auf NMDA-Rezeptoren in der Nervenzelle. Die Neuroprotektion durch Modulation der Aktivität von NMDA-Rezeptoren ist auch aus der US 6 274 633 bekannt.

Die Unterversorgung des Gehirns mit Sauerstoff führt zur Schädigung des Gehirns.

Der Erfindung liegt die Aufgabe zugrunde, ein alternatives Medikament, insbesondere ein Medikament zur Behandlung von cerebralen Störungen, bereitzustellen.

Es wurde gefunden, daß bei Verabreichung von Xenon oder Xenon-haltigen Gasen, insbesondere durch Inhalation, die Sauerstoffversorgung im Gehirn verbessert wird. Außerdem werden so kognitive, sensorische und motorische Folgezustände und Folgeerscheinungen von Sauerstoffmangel im Gehirn gelindert oder sogar geheilt.

Gegenstand der Erfindung ist die Verwendung mit den in Anspruch 1 beschriebenen Merkmalen.

Cerebrale Protektion ist definiert als Verminderung oder Verhinderung von cerebralen Funktionsstörungen unterschiedlicher Genese, vor allem aber in Folge von Perfusionsstörungen unklarer Ätiologie. Es kann das Medikament zur Behandlung von cerebralen Perfusionstörungen verwendet werden.

Vorliegende Ergebnisse deuten auf eine Verbesserung der cerebralen Perfusion durch verabreichtes Xenon. Nach vorläufigen Ergebnissen wirkt Xenon als Vasodilatator, insbesondere im kapillären Stromgebiet als kapillarer Vasodilatator. Ferner bleibt nach vorläufigen Ergebnissen die cerebrale Autoregulation erhalten oder verbessert. Das Sauerstoffangebot im Gehirn wird erhöht.

Gegenstand der Erfindung ist die Verwendung von Xenon oder einem Xenon-haltigen Gas zur Herstellung eines Medikamentes zur Behandlung von Durchblutungsstörungen im Gehirn, insbesondere zur cerebralen Vasodilatation, vorzugsweise zur Herstellung eines Medikamentes zur cerebralen kapillaren Vasodilatation, insbesondere zur Herstellung eines Medikamentes zur cerebralen kapillaren Vasodilatation im kapillären Stromgebiet.

Xenon oder ein Xenon-haltiges Gasgemisch dienen insbesondere zur Herstellung eines Medikamentes

zur Prophylaxe und/oder Therapie von kognitiven Leistungsstörungen, auch postoperativ, zur Herstellung eines Medikamentes zur Behandlung von Post Ischämie Syndrom und, zur Herstellung eines Medikamentes zur Durchblutungsförderung im Gehirn.

Xenon oder ein Xenon-haltiges Gasgemisch dienen ferner zur Herstellung eines Medikamentes zur Behandlung von kognitiver Dysfunktion, insbesondere von postoperativer kognitiver Dysfunktion nach kardio-chirurgischen Operationen, auch unter Verwendung von Herz-Lugen-Maschinen (HLM) sowie Herzunterstützungssystemen (Cardiac Assist) nach allgemeinchirurgischen Eingriffen.

Das Medikament bewirkt eine Erhöhung des cerebralen Blutflusses und der Mikrozirkulation.

Das Medikament enthält Xenon oder ein Xenon-haltiges Gasgemisch. Es besteht vorzugsweise aus gasförmigem Xenon oder einem Xenon-haltigen Gasgemisch. Das Medikament besteht beispielsweise aus Xenon-Gas, einem Gasgemisch aus Xenon und Sauerstoff oder einem Gasgemisch aus Xenon, Sauerstoff und einem Inertgas.

Das Medikament ist vorzugsweise gasförmig, insbesonderes enthält es keine festen oder flüssigen Bestandteile bei der Verabreichung, liegt also bei der Verabreichung bevorzugt als reine Gasphase vor. Das Medikament wird vorzugsweise durch Inhalation über die Lunge verabreicht. Das Medikament wird auch mittels einer Herz-Lungen-Maschine verabreicht. Das Medikament dient vorzugsweise zur Behandlung des Menschen.

Das Medikament wird in der Regel als reines gasförmiges Xenon bereitgestellt. Es kann auch als Gasgemisch bereitgestellt werden. Zum Einsatz kommt das Medikament in der Regel als ein die Atmung unterhaltendes Gasgemisch, das Xenon und Sauerstoff enthält. Solche Gasgemische werden beispielsweise in der Nottallmedizin eingesetzt, wo Gasmisch- oder Gasdosiergeräte für den mobilen Einsatz zu aufwendig sind.

Gasförmiges Xenon oder Xenon-haltige Gasgemische werden besonders vorteilhaft zur Prophylaxe eingesetzt. Die prophylaktische Verabreichung von Xenon oder Xenon-haltigen Gasgemischen erfolgt beispielsweise präoperativ, intraoperativ oder postoperativ.

Das bereitgestellte Medikament oder das direkt bei der Anwendung, insbesondere in unmittelbarer Nähe zum Patienten, hergestellte Medikament ist beispielsweise ein Gasgemisch, das 1 bis 80 Vol.-% (bezogen auf Normalbedingungen, d.h. 20° C, 1 bar absolut) Xenon enthält (z. B. Rest Sauerstoff). Das Medikament, das dem Patienten verabreicht wird, enthält Xenon in pharmakologisch oder therapeutisch wirksamer Menge, insbesondere in subanästhetisch oder anästhetisch wirksamer Menge. Vorteilhaft ist ein Medikament mit Xenon in subanästhetisch wirksamer Menge. Als subanästhetisch wirksame (subanästhetische) Mengen von Xenon sind solche Mengen oder Konzentrationen von Xenon zu verstehen, die für eine Allgemeinanästhesie nicht ausreichen. Das sind im allgemeinen Mengen bis zu 70 Vol.-% Xenon, vorzugsweise bis 65 Vol.-%, besonders bevorzugt bis 60 Vol.-%, insbesondere bis 50 Vol.-% Xenon. Reines Xenon wird dementsprechend in den genannten Konzentrationen in das Atemgas des Patienten dosiert. Das heißt das dem Patienten zugeführte Atemgas enthält beispielsweise 5 bis 60 Vol.-% , 5 bis 50 Vol.-%, 5 bis 40 Vol.-%, 5 bis 30 Vol.-% oder 5 bis 20 Vol.-% Xenon. In besonderen Fällen, z.B. bei der Prophylaxe, insbesondere bei längerer Beatmung, kann eine Dosierung von Xenon in dem Atemgas mit einer niedrigen Konzentration, beispielsweise 1 bis 35 Vol.-%, 5 bis 25 Vol.-% oder 5 bis 20 Vol.-% Xenon in dem Atemgas, vorteilhaft sein.

Die Medikamente, insbesondere gasförmigen Medikamente, enthalten vorzugsweise neben Xenon ein oder mehrere Gase oder bei Körpertemperatur und Normaldruck gasförmige Stoffe. Verwendbare Gasgemische sind beispielsweise Xenon-Sauerstoff-Gasgemische oder Gasgemische von Xenon und einem oder mehrerer Inertgase wie Stickstoff oder einem Edelgas oder Xenon-Sauerstoff-Inertgas-Gasgemische. Die Beimischung eines Gases zum Xenon kann sehr vorteilhaft sein, wenn wenig Xenon in den Körper gebracht werden soll.
Beispiele von Gasen oder Gasgemischen, die als Medikament zur cerebralen Protektion eingesetzt werden: 1.) 100 Vol.-% Xenon; 2.) 70 Vol.-% Xenon / 30 Vol.-% Sauerstoff; 3.) 65 Vol.-% Xenon / 30 Vol.-% Sauerstoff / 5 Vol.-% Stickstoff; 4.) 65 Vol.-% Xenon / 35 Vol.-% Sauerstoff; 5.) 60 Vol.-% Xenon / 30 Vol.-% Sauerstoff / 10 Vol.-% Stickstoff; 6.) 60 Vol.-% Xenon / 35 Vol.-% Sauerstoff / 5 Vol.-% Stickstoff; 7.) 60 Vol.-% Xenon / 40 Vol.-% Sauerstoff; 8.) 55 Vol.-% Xenon / 25 Vol.-% Sauerstoff / 20 Vol.-% Stickstoff; 9.) 55 Vol.-% Xenon / 30 Vol.-% Sauerstoff / 1.5 Vol.-% Stickstoff; 10.) 55 Vol.-% Xenon / 35 Vol.-% Sauerstoff / 10 Vol.-% Stickstoff; 11.) 55 Vol.-% Xenon / 40 Vol.-% Sauerstoff / 5 Vol.-% Stickstoff; 12.) 55 Vol.-% Xenon / 45 Vol.-% Sauerstoff; 13.) 50 Vol.-% Xenon / 50 Vol.-% Sauerstoff; 14.) 50 Vol.-% Xenon / 45 Vol.-% Sauerstoff / 5 Vol.-% Stickstoff; 15.) 50 Vol.-% Xenon / 40 Vol.-% Sauerstoff / 10 Vol.-% Stickstoff; 16.) 50 Vol.-% Xenon / 30 Vol.-% Sauerstoff / 20 Vol.-% Stickstoff; 17.) 50 Vol.-% Xenon / 25 Vol.-% Sauerstoff / 25 Vol.-% Stickstoff; 18.) 45 Vol.-% Xenon / 55 Vol.-% Sauerstoff; 19.) 45 Vol.-% Xenon / 50 Vol.-% Sauerstoff / 5 Vol.-% Stickstoff; 20.) 45 Vol.-% Xenon / 45 Vol.-% Sauerstoff / 10 Vol.-% Stickstoff; 21.) 45 Vol.-% Xenon / 40 Vol.-% Sauerstoff / 15 Vol.-% Stickstoff; 22.) 45 Vol.-% Xenon / 35 Vol.-% Sauerstoff / 20 Vol.-% Stickstoff; 23.) 45 Vol.-% Xenon / 30 Vol.-% Sauerstoff / 25 Vol.-% Stickstoff; 24.) 45 Vol.-% Xenon / 30 Vol.-% Sauerstoff / 25 Vol.-% Stickstoff; 25.) 40 Vol.-% Xenon / 30 Vol.-% Sauerstoff / 30 Vot.-% Stickstoff; 26.) 40 Vol.-% Xenon / 50 Vol.-% Sauerstoff / 10 Vol.-% Stickstoff; 27.) 35 Vol.-% Xenon / 25 Vol.-% Sauerstoff / 40 Vol.-% Stickstoff; 28.) 35 Vol.-% Xenon / 65 Vol.-% Sauerstoff; 29.) 30 Vol.-% Xenon / 70 Vol.-% Sauerstoff; 30.) 30 Vol.-% Xenon / 50 Vol.-% Sauerstoff / 20 Vol.-% Stickstoff; 31.) 30 Vol.-% Xenon / 30 Vol.-% Sauerstoff / 40 Vol.-% Stickstoff; 32.) 20 Vol.-% Xenon / 80 Vol.-% Sauerstoff; 33.) 20 Vol.-% Xenon / 30 Vol.-% Sauerstoff / 50 Vol.-% Stickstoff; 34.) 15 Vol.-% Xenon / 30 Vol.-% Sauerstoff / 55 Vol.-% Stickstoff; 35.) 15 Vol.-% Xenon / 50 Vol.-% Sauerstoff / 35 Vol.-% Stickstoff; 36.) 10 Vol.-% Xenon / 90 Vol.-% Sauerstoff; 37.) 10 Vol.-% Xenon / 50 Vol.-% Sauerstoff / 40 Vol.-% Stickstoff; 38.) 10 Vol.-% Xenon / 30 Vol.-% Sauerstoff / 60 Vol.-% Stickstoff; 39.) 10 Vol.-% Xenon / 25 Vol.-% Sauerstoff / 65 Vol.-% Stickstoff; 40.) 5 Vol.-% Xenon / 25 Vol.-% Sauerstoff / 70 Vol.-% Stickstoff; 41.) 5 Vol.-% Xenon / 30 Vol.-% Sauerstoff / 65 Vol.-% Stickstoff; 42.) 5 Vol.-% Xenon / 50 Vol.-% Sauerstoff / 45 Vol.-% Stickstoff; 43.) 5 Vol.-% Xenon / 30 Vol.-% Sauerstoff / 65 Vol.-% Stickstoff; 44.) 5 Vol.-% Xenon / 95 Vol.-% Sauerstoff; 45.) 1 Vol.-% Xenon / 99 Vol.-% Sauerstoff; 46.) 1 Vol.-% Xenon / 30 Vol.-% Sauerstoff / 69 Vol.-% Stickstoff; 47.) 1 Vol.-% Xenon / 25 Vol.-% Sauerstoff / 74 Vol.-% Stickstoff.

Besonders vorteilhaft wird das Medikament, , in der Intensivmedizin eingesetzt, insbesondere wenn das Medikament über einen längeren Zeitraum verabreicht werden muß, beispielsweise bei der Langzeitbeatmung. Hier hat das Medikament den besonderen Vorteil, nach derzeitigem Kenntnisstand keine Nebenwirkungen zu haben, da das Medikament selbst nicht vom Körper metabolisiet wird. So bilden sich im Körper bei Verwendung von Xenon oder Xenon-haltigen Gasen als Medikament keine Metabolite im Körper und es findet im Körper keine Anreicherung des Medikamentes statt.

Xenon wird insbesondere bei der Langzeitbeatmung und bei der Prophylaxe vorteilhaft in subanästhetisch wirksamen Konzentrationen in einem atembaren Gas (Atemgas) verabreicht. Insbesondere bei der Langzeitbeatmung ist die Verabreichung von atembaren Gasen mit einem Gehalt von 5 bis 45 Vol.-% Xenon, vorzugsweise 5 bis 40 Vol.-% Xenon, vorteilhaft. Bei der Langzeitbeatmung hat das atembare Gas beispielsweise einen Gehalt von 20 bis 30 Vol.-% Sauerstoff, wobei Sauerstoffgehalt bei Bedarf zeitweise z. B. auf 30 bis 95 Vol.-% Sauerstoff erhöht werden kann. Das restliche Gas in dem atembaren Gas besteht in der Regel aus Stickstoff oder einem anderen Inertgas.

Das eingesetzte Xenon-Gas hat im allegemeinen die natürliche Isotopenzusammensetzung. Die Isotopenzusammensetzung des Xenons kann sich von der natürlichen Isotopenzusammensetzung unterscheiden, insbesondere bei Verwendung zu diagnostischen Zwecken. Das Xenon-Gas wird vorzugsweise in hoher Reinheit, wie für medizinische Gase üblich, eingesetzt. Das Xenon-Gas dient vorzugsweise als reines Gas oder im Gemisch mit anderen Gasen zur Herstellung eines gasförmigen Medikamentes für die genannten Anwendungen.

Gasförmiges Xenon (reines Xenon) wird im allgemeinen als komprimiertes Gas in Druckgasbehältern wie Druckgasflaschen oder Druckdosen bereitgestellt. Auch können Xenon-haltige Gasgemische in Druckgasbehältern bereitgestellt werden. Das gasförmige Medikament kann auch in einem Behälter als verflüssigtes Gas oder Gasgemisch oder in kälteverfestigter Form bereitgestellt werden.

Das Medikament wird in der Regel mit einem Beatmungsgerät mit einer Gasdosiereinheit oder mit einem Anästhesiegerät verabreicht. Das Arzneimittel wird vorteilhaft direkt zur Anwendung aus den reinen Gasen hergestellt, beispielsweise durch Zusammenmischen von Xenon, Sauerstoff und gegebenenfalls einem Inertgas (z. B. mit Hilfe eines Anästhesiegerätes) in unmittelbarer Nähe zum Patienten.

Das Medikament wird in der Regel als trockenes, feuchtes Gas oder wasserdampfgesättigtes Gas dem Patienten verabreicht.

Vorteilhaft ist ferner die Kombination des Xenon-haltigen Medikaments mit einem Medikament, das eine NO-Quelle enthält, zur Behandlung von cerebralen Durchblutungsstörungen.

Das Medikament, enthält somit Xenon und eine NO-Quelle oder ein Xenon-haltiges Gasgemisch und eine NO-Quelle, wobei Xenon und NO-Quelle in pharmakologisch wirksamer Konzentration enthalten sind.

Gegenstand der Erfindung ist insbesondere die Verwendung eines Medikaments bestehend aus einem Inhalationsmedikament, enthaltend Xenon oder ein xenonhaltiges Gas, und ein vorzugsweise oral, inhalativ oder parenteral verabreichtes Medikament, das eine NO-Quelle enthält, als Kombinationspräparat zur gleichzeitigen, getrennten oder zeitlich abgestuften Anwendung. Zur Behandlung von Durchblutungsstörungen im Gehirn.

Ein weiterer Gegenstand der Erfindung is insbesondere Verwendung von Xenon und einer NO-Quelle oder einem xenonhaltigen Gas und einer NO-Quelle zur Behandlung cerebraler Perfusionsstörungen.

Das als Kombinationspräparat dienende Medikament besteht vorzugsweise aus einem Inhalationsmedikament, enthaltend Xenon oder ein xenonhaltiges Gas, und einem oral, inhalativ (z.B. als Aerosol) oder parenteral verabreichten Medikament, das eine NO-Quelle enthält.

Eine Stickstoffmonoxid-Quelle (NO-Quelle) ist NO (Stickstoffmonoxid), ein NO enthaltendes Gas oder Gasgemisch oder vorzugsweise eine Substanz oder Präparation, die Stickstoffmonoxid (NO) freigibt, enthält, die körpereigene NO-Bildung anregt oder den Abbau von NO im Körper hemmt. Eine Stickstoffmonoxid-Quelle sind insbesondere NO-abgebende und/oder NO-bildende Verbindungen.

NO-Quellen und insbesondere NO-abgebende Verbindungen sind in der DE 691 27 756 T2 (dort z.B. Seite 8, Zeile 7, bis Seite 9, Ende des zweiten Absatzes) beschrieben, worauf hiermit Bezug genommen wird. NO-abgebende Verbindungen sind z.B. S-Nitroso-N-acetylpenicillamin (SNAP), S-Nitrosocystein, Nitroprussid, Nitrosoguanidin, Glyceroltrinitrat, Isoamylnitrit, anorganisches Nitrit, Azid oder Hydroxylamin. Die NO-abgebenden Verbindungen werden beispielsweise als Aerosol durch Inhalation in die Lunge eingebracht, wie in der DE 691 27 756 T2 beschrieben ist und worauf hiermit Bezug genommen wird.

Wird eine Erhöhung des NO-Spiegels im Gehirn bei der Verabreichung von NO-abgebenden Verbindungen über das Blut bezweckt, so kann dies durch die Blut-Him-Schranke behindert werden. Dieses Problem wird nach der WO 00/56328 durch Verabreichung eines die körpereigene NO-Bildung stimulierenden Agens wie L-Arginin umgangen. Stoffe, die die körpereigene NO-Bildung erhöhen, werden als NO-bildende Substanzen angesehen. Die körpereigene NO-Bildung anregende Substanzen werden vorteilhaft in Kombination mit NO-abgebenden Verbindungen eingesetzt. Die vorhergehende oder gleichzeitige Verabreichung von einer oder mehreren die körpereigene NO-Bildung anregenden Substanzen verbessern die Zuführbarkeit der NO-abgebenden Verbindungen in das Gehirn, was zu einer verbesserten Wirksamkeit der NO-abgebenden Verbindungen in dem Gehirn führt. Eine oder mehrere die körpereigene NO-Bildung anregende Substanzen, eine oder mehrere NO-abgebenden Verbindungen und Xenon oder ein Xenon-haltiges Gas werden somit vorteilhaft zeitlich getrennt oder gleichzeitig verabreicht. Beispielsweise wird mit der Verabreichung einer die körpereigene NO-Bildung anregende Substanz begonnen und in einem weiteren Schritt werden NO-abgebende Verbindung und Xenon verabreicht. Es kann auch vorteilhaft sein, mit der inhalativen Verabreichung von Xenon zu beginnen und in einem weiteren Schritt eine die körpereigene NO-Bildung anregende Substanz und eine NO-abgebende Verbindung, gleichzeitig oder zeitlich getrennt zu verabreichen.

Das Kombinationsmedikament enthält Xenon und mindestens eine NO-Quelle in therapeutisch wirksamer Menge. Das Medikament enthält Xenon z.B. in subanästhetisch oder anästhetisch wirksamer Menge. Die Dosierung von die körpereigene NO-Bildung anregenden Substanzen wird z.B. in der WO 00/56328 beschrieben, worauf hiermit Bezug genommen wird. Die Dosierung von NO-abgebenden Verbindungen ist für die Verabreichung als Aerosol in der US 5 485 827 beschrieben, worauf hiermit Bezug genommen wird.

Xenon oder Xenon-haltige Gase und eine NO-Quelle dienen zur Herstellung eines Medikamentes zur Behandlung von Durchblutungsstörungen im Gehirn, insbesondere zur Herstellung eines Medikamentes zur Behandlung von cerebraler Perfusionsstörung, zur Herstellung eines Medikamentes zur Prophylaxe und/oder Therapie von kognitiven Leistungsstörungen, auch postoperativ, zur Herstellung eines Medikamentes zur Behandlung von Post Ischämie Syndrom, und zur Herstellung eines Medikamentes zur Durchblutungsförderung im Gehirn.

Beispielsweise bei einem Schlaganfall wird das Medikament vorteilhaft in folgender Weise eingesetzt. Zunächst wird ein Xenon-haltiges Gas verabreicht, z.B. in subanästhetisch und sedierend wirksamer Menge. In einer nächsten Phase der zeitlich abgestuften Verabreichung des Medikamentes wird vorteilhaft eine gasförmige, flüssige oder feste Präparation mit einer NO-Quelle (ein oder mehrere Stoffe zur Erhöhung des NO-Spiegels im Gehirn) oder eine Präparation mit einer NO-Quelle in Kombination mit Xenon oder einem Xenon-haltigen Gas verabreicht.

Xenon oder ein Xenon-haltiges Gasgemisch und eine NO-Quelle dienen ferner zur Herstellung eines Medikamentes zur Prophylaxe und/oder Therapie von Kognitiven Leistungsstörungen, auch postoperativ.

Zum Einsatz kommt das Kombinationsmedikament in der Regel als Kombination einer Komponente (z.B. als Inhalationsmedikament) mit Xenon und einer weiteren Komponente mit einer NO-Quelle, z.B. einem parenteral, inhalativ oder oral verabreichten Medikament mit mindestens einer NO-Quelle. Das Medikament enthält Xenon in einer pharmakologisch oder therapeutisch wirksamen Menge, z.B. in subanästhetisch oder anästhetisch wirksamer Menge. Das Medikament enthält die NO-Quelle in einer pharmakologisch oder therapeutisch wirksamen Menge.

Das Kombinationsmedikament besteht beispielsweise aus Xenon, einem Inertgas und einer NO-Quelle oder aus Xenon, Sauerstoff, einem Inertgas und einer NO-Quelle.

## Patentansprüche

1. Verwendung von Xenon oder eines Xenon-haltigen Gasgemisches zur Herstellung eines Medikamentes zur Behandlung von Durchblutungsstörungen im Gehirn.

2. Die Verwendung nach Anspruch 1, **dadurch gekennzeichnet, dass** Xenon in dem Medikament in therapeutisch wirksamer Menge enthalten ist.

3. Die Verwendung nach Anspruch 1 oder 2, **dadurch gekennzeichnet, dass** das Medikament zur Behandlung von Durchblutungsstörungen als Rest Sauerstoff oder Sauerstoff und ein Inertgas enthält.

4. Die Verwendung nach einem der Ansprüche 1 bis 3, **dadurch gekennzeichnet, dass** das Medikament als Kombinationspräparat mit einer gasförmigen, flüssigen oder festen Präparation, enthaltend eine NO-Quelle, zur gleichzeitigen, getrennten oder zeitlich abgestuften Anwendung, verwendet wird.

5. Die Verwendung nach einem der Ansprüche 1 bis 4, **dadurch gekennzeichnet, dass** das Medikament Xenon und eine NO-Quelle oder ein Xenonhaltiges Gasgemisch und eine NO-Quelle enthält, wobei Xenon und NO-Quelle in therapeutisch wirksamer Menge enthalten sind.

6. Die Verwendung nach einem der Ansprüche 1 bis 5, **dadurch gekennzeichnet, dass** das Medikament zur cerebralen Vasodilation oder zur Behandlung, Therapie oder Prophylaxe von kognitiven Leistungsstörungen oder kognitiver Dysfunktion verwendet wird.

7. Die Verwendung nach einem der Ansprüche 1 bis 6 **dadurch gekennzeichnet, dass** das Medikament zur Behandlung cerebraler Perfusionsstörungen verwendet wird.

8. Die Verwendung nach einem der Ansprüche 1 bis 7, **dadurch gekennzeichnet, dass** das Medikament zur Behandlung von Post Ischämie Syndromen verwendet wird.

9. Die Verwendung nach einem der Ansprüche 1 bis 8, **dadurch gekennzeichnet, dass** das Medikament zur Durchblutungsförderung im Gehirn verwendet wird.

10. Die Verwendung nach einem der Ansprüche 1 bis 9, **dadurch gekennzeichnet, dass** das Medikament zur cerebralen Vasodilation verwendet wird.

## Claims

1. The use of Xenon or a mixture of gas containing Xenon for the preparation of a medicament for the treatment of dysfunction of the blood circulation in the brain

2. The use according to claim 1, **characterized in that** Xenon is contained in the medicament in a therapeutically effective amount.

3. The use according to claim 1 or 2, **characterized in that** the medicament for the treatment of dysfunction of the blood circulation contains as residue oxygen or oxygen and an inert gas.

4. The use according to anyone of claims 1 to 3, **characterized in that** the medicament is used simultaneously, separately or sequentially as a combination preparation with a gaseous, liquid or solid preparation, containing a NO-source.

5. The use according to anyone of claims 1 to 4, **characterized in that** the medicament contains Xenon and a NO-source or a gas mixture containing Xenon and a NO-source, wherein Xenon and the NO-source are contained in a therapeutically effective amount.

6. The use according to anyone of claims 1 to 5, **characterized in that** the medicament is used for cerebral vasodilatation or for the treatment, therapy or prophylaxis of dysfunction of cognitive performance or cognitive dysfunction.

7. The use according to anyone of claims 1 to 6, **characterized in that** the medicament is used for the treatment of cerebral perfusion dysfunction.

8. The use according to anyone of claims 1 to 7, **characterized in that** the medicament is used for the treatment of post-ischemia syndrome.

9. The use according to anyone of claims 1 to 8, **characterized in that** the medicament is used to improve the blood perfusion in the brain.

10. The use according to anyone of claims 1 to 9, **characterized in that** the medicament is used for cerebral vasodilatation.

## Revendications

1. Utilisation de Xénon ou d'un mélange de gaz contenant du Xénon pour la préparation d'un médicament destiné au traitement de dysfonction de la circulation sanguine dans le cerveau.

2. L'utilisation selon la revendication 1, **caractérisée en ce que** le Xénon est contenu dans le médicament en quantités à efficacité thérapeutique.

3. L'utilisation selon la revendication 1 ou 2, **caractérisée en ce que** pour traiter la dysfonction de la circulation sanguine le médicament contient comme résidu de l'oxygène ou de l'oxygène et un gaz inerte.

4. L'utilisation selon l'une quelconque des revendications 1 à 3, **caractérisée en ce que** le médicament est utilisé comme préparation de combinaison avec une préparation gazéiforme, liquide ou solide, contenant une source de NO pour l'utilisation simultanée, séparée ou temporairement dégradée.

5. L'utilisation selon l'une quelconque des revendications 1 à 4, **caractérisée en ce que** le médicament contient du Xénon et une source de NO ou un mélange de gaz contenant du Xénon et une source de NO, le Xénon et la source de NO étant contenus en quantités à efficacité thérapeutique.

6. L'utilisation selon l'une quelconque des revendications 1 à 5, **caractérisée en ce que** le médicament est utilisé pour la vasodilatation cérébrale ou pour le traitement, la thérapie ou la prophylaxie de la dysfonction de la performance cognitive ou de la dysfonction cognitive.

7. L'utilisation selon l'une quelconque des revendications 1 à 6, **caractérisée en ce que** le médicament est utilisé pour le traitement de la dysfonction de la circulation cérébrale.

8. L'utilisation selon l'une quelconque des revendications 1 à 7, **caractérisée en ce que** le médicament est utilisé pour le traitement de syndromes post-ischémiques.

9. L'utilisation selon l'une quelconque des revendications 1 à 8, **caractérisée en ce que** le médicament est utilisé pour favoriser la circulation sanguine dans le cerveau.

10. L'utilisation selon l'une quelconque des revendications 1 à 9, **caractérisée en ce que** le médicament est utilisé pour la vasodilatation cérébrale.
